# EUROPEAN PATENT APPLICATION

(11) **EP 2 418 279 A1**
(43) Date of publication of application: **15.02.2012**
(21) Application number: 10305878.0
(22) Date of filing: 10.08.2010
(51) Int. Cl.: C12N 15/113, A61K 31/711, A61P 9/00, A61P 27/02

(54) **Injectable pharmaceutical composition for preventing, stabilising and/or inhibiting pathological neovascularization-related conditions**

(71) Applicant: Gene Signal International SA, 1015 Lausanne (CH)
(72) Inventor: Al Mahmood, Salman, 75014 Paris (FR); Colin, Sylvie, 75014 Paris (FR); Conduzorgues, Jean-Pascal, 34070 Montpellier (FR); Viaud, Eric, 1010 Lausanne (CH)
(74) Representative: Icosa

(57) **Abstract**

This invention relates to a pharmaceutical composition for the treatment and/or prevention of at least one pathological neovascularization-related conditions of the interior of the eye, said composition comprising a therapeutically effective amount of an antisens oligonucleotide having the sequence SEQ ID NO: 1,
**5'-TCTCCGGAGGGCTCGCCATGCTGCT-3'**
or any function conservative sequence comprising from 9 to 30 nucleotides that has 75%, 80%, 85%, 90%, 95% or more than 95%, 96%, 97%, 98%, 99% of identity compared to SEQ ID NO: 1 and that conserves the capacity of inhibiting IRS-1 gene expression as SEQ ID NO: 1,
and said composition being administered to a subject in need thereof, by intraocular route; this invention also relates to a method for treating a pathological neovascularization-related condition of the interior of the eye in a subject in need thereof comprising administering to the subject a therapeutically effective amount of said pharmaceutical composition.

## Description

### Field of the invention

This invention relates to the treatment of pathological neovascularization-related conditions, especially in the field of ophthalmology. In particular, this invention relates to an injectable pharmaceutical composition containing an antisense oligonucleotide GS-101, capable of inhibiting the expression of the insulin receptor substrate-1 (IRS-1), to prevent and/or treat ophthalmic pathological neovascularization-related conditions.

### Background of the invention

Angiogenesis is a fundamental process by means of which new blood vessels are formed. This process is essential in multiple normal physiological phenomena such as reproduction, development and even wound healing.

The formation of neovessels by endothelial cells, involves the migration, growth and differentiation of endothelial cells. Regulation of these biological phenomena is linked to Insulin receptor substrate 1 (IRS-1), which is a cytoplasmic docking protein that functions as an essential signalling intermediate downstream of activated cell surface receptors, including insulin, insulin-like growth factor 1 (IGF-1), prolactin, growth hormone (GH), vascular endothelial growth factor (VEGF) receptors, members of the integrin receptor family, and select cytokine receptors.

Even though neovascularisation may be a normal physiological process, pathological neovascularisation is a critical situation in a number of diseases, especially in ophthalmology.

In ophthalmology, pathologies of the posterior segment of the eye, and in particular retinal pathologies, are some of the more disabling pathologies of modern society. Numbered among these pathologies are those characterized by abnormal neovascularization of the retina, iris and choroid, with consequent formation of dysfunctional neovessels which can cause leakage or haemorrhages, or can be associated with retinal edema, retinal/vitreous haemorrhage or retinal detachment resulting in the decline of visual acuity (Survey of Ophthalmology, Jan. 2007, Vol. 52, S1, S3-S19). Ocular pathological neovascularization is also known as one of the leading causes of blindness in humans and is found in diverse eye diseases (Bradley, et al., 2007, Angiogenesis 10:141-8; Chen and Smith, 2007, Angiogenesis 10:133-40; Friedlander et al., 2007, Angiogenesis 10:89-101).

Among the therapies currently being practiced to treat ocular posterior segment disorders, such as uveitis, macular degeneration, macular edema and the like, is intravitreal injection of corticosteroids.

However, corticosteroids have well-known drawbacks, and there is still a need for new therapies for treating the disorders, conditions or diseases of the interior of the eye, which are conditions related to pathological neovascularization.

This invention brings a solution : it was now found that a specific oligonucleotide, called GS-101, already known in topical administration for its capacity of inhibiting IRS-1 gene expression (see for example EP 1 409 672) was of particular interest, when administered intraocularly, for the treatment of pathologies of the interior of the eye related to pathological neovascularization. The intraocular route brings the further advantage to make it possible to administrate a therapeutically effective, but low amount of active ingredient, to the patient. It also have a further advantage to control the exact administered amount, and to avoid any non-compliance to the treatment due to patient.

### Summary of the invention

The invention thus proposes a composition for treating or for use in treating ocular disorders of the interior of the eye, linked to pathological neovascularization, wherein said composition comprises a therapeutically effective amount of GS-101 and said composition is administered to a subject in need thereof, by intraocular route.

According to the invention, the composition is formulated in a form suitable for intraocular injections. According to a first embodiment, the composition is an aqueous solution. According to a second embodiment the composition is within an implant. According to a third embodiment, the composition is associated with a sustained delivery sytem, composition or device. According to a fourth embodiment, the composition may include GS 101 and a polymeric agent.

According to the invention, GS-101 is an antisens oligonucleotide having the sequence
SEQ ID NO: 1, **5'-TCTCCGGAGGGCTCGCCATGCTGCT-3'**
or any function conservative sequence comprising from 9 to 30 nucleotides that has 75%, 80%, 85%, 90%, 95% or more than 95%, 96%, 97%, 98%, 99% of identity compared to SEQ ID NO: 1 and that conserves the capacity of inhibiting pathological neovascularization as SEQ ID NO: 1.

An example of a function conservative sequence of SEQ ID NO: 1 is SEQ ID NO: 2 (5'-TATCCGGAGGGCTCGCCATGCTGCT-3'). Other examples of a function conservative sequence of SEQ ID NO: 1 are the following sequences:
5'-TCTCCGGAGGGCTCGCCATGCTGC-3' (SEQ ID NO: 3)
5'-TCTCCGGAGGGCTCGCCATGCTG-3' (SEQ ID NO: 4)
5'-TCTCCGGAGGGCTCGCCATGCT-3' (SEQ ID NO: 5)
5'-TCTCCGGAGGGCTCGCCATGC-3' (SEQ ID NO: 6)
5'-TCTCCGGAGGGCTCGCCATG-3' (SEQ ID NO: 7)
5'-TCTCCGGAGGGCTCGCCAT-3' (SEQ ID NO: 8)
5'-CTCCGGAGGGCTCGCCATGCTGCT-3' (SEQ ID NO: 9)
5'-TCCGGAGGGCTCGCCATGCTGCT-3' (SEQ ID NO: 10)
5'-CCGGAGGGCTCGCCATGCTGCT-3' (SEQ ID NO: 11)
5'-CGGAGGGCTCGCCATGCTGCT-3' (SEQ ID NO: 12)
5'-GGAGGGCTCGCCATGCTGCT-3' (SEQ ID NO: 13)
5'-GAGGGCTCGCCATGCTGCT-3' (SEQ ID NO: 14)
5'-AGGGCTCGCCATGCTGCT-3' (SEQ ID NO: 15)
5'-GGCTCGCCATGCTGCT-3' (SEQ ID NO: 16)
5'-GCTCGCCATGCTGCT-3' (SEQ ID NO: 17)
5'-CTCGCCATGCTGCT-3' (SEQ ID NO: 18)
5'-TCGCCATGCTGCT-3' (SEQ ID NO: 19)
5'-CGCCATGCTGCT-3' (SEQ ID NO: 20)
According to an embodiment, said function conservative sequence comprising 9 to 30 nucleotides may be a sequence comprising SEQ ID NO: 1 or SEQ ID NO: 2 between other nucleic acids in C-terminal and N-terminal. Said function conservative sequence may also be a 9 to 12 contiguous nucleotides fragment of SEQ ID NO: 1 or SEQ ID NO: 2.

The pharmaceutical composition may include GS-101 as described above, or a function conservative sequence thereof, as described above, in association with any pharmaceutically acceptable excipients for intraocular route.

The present invention also relates to a method for treating a pathological neovascularization-related condition of the interior of the eye in a subject in need thereof comprising administering to the subject a therapeutically effective amount of a composition comprising GS-101 as described above, or a function conservative sequence thereof, as described above, in association with a pharmaceutically acceptable excipients, and said composition is administered by intraocular route.

According to an embodiment, GS-101 as described above, or a function conservative sequence thereof, is present in the composition of the invention in a concentration of about 0.01 mg/ml to about 100 mg/ml, preferably 0.1 mg/ml to 80 mg/ml, more preferably 6 to 60 mg/ml.

According to an embodiment, the volume of the injected composition per administration ranges from 1-500 µl, preferably from about 5-400 µl, more preferably from 5-300 µl. According to an embodiment, the volume of the injected composition is of about 50 µl.

In one embodiment, the pharmaceutical composition is such that the intraocular administration is intravitreal administration.

According to an embodiment, the composition is injected at most once every four weeks, even more preferably once every eight weeks. According to an embodiment, the patient is treated with one injection every four weeks during two to five, preferably three months.

According to an embodiment, the duration of the treatment is of three to six months months. The treatment may be renewed if a loss of visual acuity is observed in the patient.

In one embodiment of the invention, said pharmaceutical composition as described here above is packaged in the form of unit dose.

In another embodiment, said unit dose is a disposable syringe.

Advantageously, said unit dose is sterile.

### Definitions

According to this invention, the following terms have the following meanings:
**"About"** means plus or minus ten percent of the number, parameter or characteristic so qualified.
**"Interior of the eye"** means any area located within the eyeball, including the anterior and posterior segment of the eye, and which generally includes, but is not limited to, any functional (e.g., for vision) or structural tissues found within the eyeball, or tissues or cellular layers that partly or completely line the interior of the eyeball. Specific examples of areas include the anterior chamber, the posterior chamber, the vitreous cavity, the choroid, the macula, and the retina, and blood vessels and nerves which vascularize or innervate a posterior ocular region or site. According to a preferred embodiment, interior of the eye means the posterior segment of the eye, including the posterior chamber, the vitreous cavity, the choroid, the macula, and the retina, and blood vessels and nerves which vascularize or innervate a posterior ocular region or site.
**"Conditions related to pathological neovascularization" or "pathological neovascularization-related conditions"** are disease where undesired neovascularization is present, and includes the following: uveitis, choroiditis, retinochoroiditis, chorioretinitis, retinal degeneration, AMD, retinal detachment, retinal neovascularization, proliferative vitreoretinopathy, retinopathy of prematurity (ROP), diabetic retinopathy, posterior segment trauma, retinal vascular pathologies, endophthalmitis, macular edema, diabetic retinopathy, inflammatory pathologies of the retina, systemic pathologies with implications for the retina, possibly in combination with other therapies for the treatment of the same pathologies. Especially, the invention aims at addressing blood and lymph neovascularization.
**"Therapeutically effective amount"** means level or amount of agent that is aimed at, without causing significant negative or adverse side effects to the target, (1) delaying or preventing the onset of a disease, disorder, or condition related to pathological neovascularization; (2) slowing down or stopping the progression, aggravation, or deterioration of one or more symptoms of the disease, disorder, or condition related to pathological neovascularization; (3) bringing about ameliorations of the symptoms of the disease, disorder, or condition related to pathological neovascularization; (4) reducing the severity or incidence of the disease, disorder, or condition related to pathological neovascularization; or (5) curing the disease, disorder, or condition related to pathological neovascularization. A therapeutically effective amount may be administered prior to the onset of the disease, disorder, or condition related to pathological neovascularization, for a prophylactic or preventive action. Alternatively or additionally, the therapeutically effective amount may be administered after initiation of the disease, disorder, or condition related to pathological neovascularization, for a therapeutic action. In one embodiment, the target is the interior of the eye ; in this embodiment, a therapeutically effective amount of GS 101, is an amount that is effective in reducing at least one symptom of a pathological neovascularization-related condition of the interior of the eye.
**"Intraocularly"** means by intraocular route of administration. Preferred intraocular route is intravitreal injection. According to the present invention, the intraocular route is of specific interest, in that it results in administering a rather low, and therapeutically accurate dose of active ingredient (i.e. GS-101 or function conservative sequence thereof) and result in an optimal treatment.
**"Subject"** refers to a mammal, preferably a human, but also can refer to animals, such as for example pets.

### Description of the figures

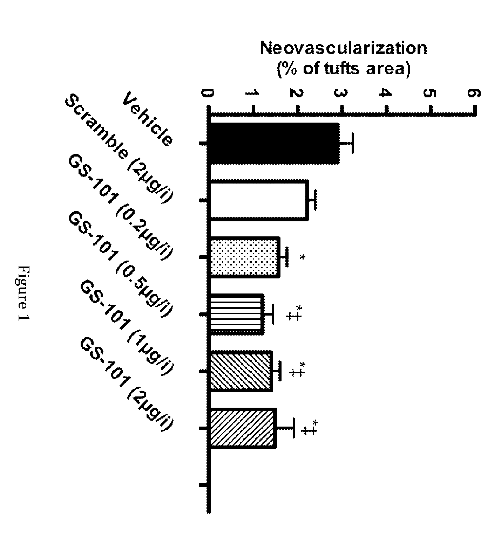

**Figure 1**. Effects of two intraocular injections of GS-101 or its vehicle (NaCl) on neovascularization of the retina following OIR. Results are mean±SEM. *: P<0.01 compared to vehicle ; ‡: P<0.05 compared to Scramble. (Scramble is an oligonucleotide with same bases as GS101, but not with the same sequence, from wich was designed the term "scramble").

### Examples

### Experimental approaches

The procedures and protocols were approved by our institutional review board and were performed in accordance with our institutional guidelines and the *Guide for the Care and Use of Laboratory Animals* of France and Canada. Animals were kept under standard conditions with free access to food and water (24°C; 12:12hr light/dark cycle).

### PHARMACOKINETIC ANALYSIS

Animal study: Male adult New Zealand albino rabbits (n=30, 4 months old, weight range: 2.138 to 3.339 Kg; from the Bergerie de la combe aux loups, Boisset St Priest, France) were used.

### OXYGEN-INDUCED RETINOPATHY IN RATS

Oxygen-induced retinopathy (OIR) rat model was used as previously described (Dorfman A, Dembinska O, Chemtob S, Lachapelle P. Early manifestations of postnatal hyperoxia on the retinal structure and function of the neonatal rat. Invest Ophthalmol Vis Sci 2008;49:458-466). Newborn litters of Sprague-Dawley rats (Charles River Laboratories, St-Constant, Quebec, Canada) were exposed to 80% oxygen immediately after birth (mixture of medical grade 100% O₂ and room air measured with an oxygen meter; MaxO₂ Ceramatec, model OM25-ME; Medicana Inc., Montreal, Quebec, Canada). Briefly, exposure to hyperoxia (80% O₂) persisted from birth until postnatal day 14 for 22.5 hours daily, interrupted with three intervals of 30 minutes' duration under normoxic conditions (21% O₂). After hyperoxic exposure, animals were assigned at P14 to the following experiment, animals were treated at P14 and P16 by an intravitreal injection of 1 µl in one eye of either the vehicle alone (sterile NaCl 0.9%) (n=8), or containing either a scramble GS-101 oligonucleotide (2 mg/ml, 2 µg delivered, n=4), or GS-101 (SEQ ID NO: 2) at the concentration of 0.5 mg/ml (0.5 µg delivered, n=7), 1 (1 µg delivered, n=8), and 2 (2 µg delivered, n=8). Before the injection, rats were anesthetized with halothane (~2.5%) and injected with a 10 µl-Hamilton syringe attached to a glass capillary of approximately 60 gauge. During the treatment period, rats were maintained in a cyclic lighting environment (80 lux; 12 hours dark/12 hours light). Finally, mothers of the litters were alternated between normoxic and hyperoxic conditions every 24 hours so that pulmonary complications known to arise in adult rats raised in a hyperoxic environment could be avoided. All animals were then euthanized at P18: the eyes were enucleated, the anterior segments dissected, and the eyecups fixed overnight in 4% formalin. Doses detailed above are based on the estimated eye volumes (~25 µl in third postnatal week vs. ~50 µl in adult).

### DEVELOPMENTAL RETINAL VASCULARIZATION IN NEWBORN RATS

Under halothane anesthesia, 1 µl in one eye of either the vehicle alone (sterile NaCl 0.9%) (n=6), or containing GS-101 at the concentration of 0.5 mg/ml (0.5 µg delivered, n=8) was injected at P1 and P3. Rat pup eye volume is approximately 0.3% of that of human adult.

### RETINAL FLATMOUNTS

The retinas were subsequently isolated, and flatmounts were prepared for staining with adenosine diphosphatase (ADPase). Specimens were mounted and photographed (40x, Axiophot microscope; Carl Zeiss Meditec, GmbH, Oberkochen, Germany). For the developmental protocol, retinal vascularization area and density have been evaluated using ImagePro Plus 4.5 (Media Cybernetics, Silver Spring, MD). For the OIR protocol, the severity of retinopathy has been assessed using a retinal scoring system which evaluates the following criteria: blood vessel growth, blood vessel tufts, extra-retinal neovascularization, central vasoconstriction, retinal hemorrhage, and blood vessel tortuosity.²² In addition, vascular tufts *per se* have been evaluated on retinal flatmounts.

### STATISTICAL ANALYSIS

Statistical analyses were made by ANOVA using the Dunnett posttest (GraphPad Software Inc, San Diego, CA, USA). P<0.05 were considered significant.

To determine the effective concentration of GS-101 required in the posterior chamber to inhibit retinal neovascularization in rats with OIR, GS-101 was injected in the eye. As shown by Figure 1, a dose of 0.5 µg reduced pathological neovascularization. Considering the volume of the eye (25 µl), this suggests that a concentration of 20 µg/ml is required to reduce retinal neovascularization in the rat model of OIR

### RESULTS

**Table 1. Effects of intra-ocular injection of GS-101 on normal vascularization and its density in the retina during development in newborn rats.**

| | **Vehicle** | | **GS-101 0.5 µg/l** | |
|---|---|---|---|---|
| | Vase Area (%) | Density (%) | Vase Area (%) | Density (%) |
| **Moy** | 53.60 | 39.85 | 51.87 | 34.23 |
| | | | | |
| **SEM** | 1.77 | 1.66 | 2.76 | 2.46 |
| | | | | |
| **N** | 6 | 7 | 8 | 8 |

| | | | | |
|---|---|---|---|---|
| Vase Area: vascular area. | | | | |

As demonstrated in table 1, intraocular injections of GS-101 does not alter the normal evolution of the vascularization of the retina at concentration that have been shown to be effective in the rat model of OIR (Fig. 1). Hence, GS-101 confirms its safety profile.

## Claims

1. Pharmaceutical composition for use in the treatment and/or prevention of at least one pathological neovascularization-related conditions of the interior of the eye, said composition comprising a therapeutically effective amount of an antisens oligonucleotide having the sequence SEQ ID NO: 1,
**5'-TCTCCGGAGGGCTCGCCATGCTGCT-3'**
or any function conservative sequence comprising from 9 to 30 nucleotides that has 75%, 80%, 85%, 90%, 95% or more than 95%, 96%, 97%, 98%, 99% of identity compared to SEQ ID NO: 1 and that conserves the capacity of inhibiting IRS-1 gene expression as SEQ ID NO: 1, and said composition being administered to a subject in need thereof by intraocular route.

2. Pharmaceutical composition according to Claim **1,** where the function conservative sequence of SEQ ID NO: 1 is 5'-TATCCGGAGGGCTCGCCATGCTGCT-3' (SEQ ID NO: 2).

3. Pharmaceutical composition according to Claim **1,** where the function conservative sequence of SEQ ID NO: 1 is selected from the group consisting of:
5'-TCTCCGGAGGGCTCGCCATGCTGC-3' (SEQ ID NO: 3)
5'-TCTCCGGAGGGCTCGCCATGCTG-3' (SEQ ID NO: 4)
5'-TCTCCGGAGGGCTCGCCATGCT-3' (SEQ ID NO: 5)
5'-TCTCCGGAGGGCTCGCCATGC-3' (SEQ ID NO: 6)
5'-TCTCCGGAGGGCTCGCCATG-3' (SEQ ID NO: 7)
5'-TCTCCGGAGGGCTCGCCAT-3' (SEQ ID NO: 8)
5'-CTCCGGAGGGCTCGCCATGCTGCT-3' (SEQ ID NO: 9)
5'-TCCGGAGGGCTCGCCATGCTGCT-3' (SEQ ID NO: 10)
5'-CCGGAGGGCTCGCCATGCTGCT-3' (SEQ ID NO: 11)
5'-CGGAGGGCTCGCCATGCTGCT-3' (SEQ ID NO: 12)
5'-GGAGGGCTCGCCATGCTGCT-3' (SEQ ID NO: 13)
5'-GAGGGCTCGCCATGCTGCT-3' (SEQ ID NO: 14)
5'-AGGGCTCGCCATGCTGCT-3' (SEQ ID NO: 15)
5'-GGCTCGCCATGCTGCT-3' (SEQ ID NO: 16)
5'-GCTCGCCATGCTGCT-3' (SEQ ID NO: 17)
5'-CTCGCCATGCTGCT-3' (SEQ ID NO: 18)
5'-TCGCCATGCTGCT-3' (SEQ ID NO: 19)
5'-CGCCATGCTGCT-3' (SEQ ID NO: 20)

4. Pharmaceutical composition according to anyone of Claims 1 to 3, wherein the oligonucleotide is in a concentration of about 0.01 mg/ml to about 100 mg/ml.

5. Pharmaceutical composition according to anyone of Claims **1** to **4,** wherein the volume of the injected composition per administration ranges from 1 to 500 µl.

6. Pharmaceutical composition according to anyone of Claims **1** to **5,** wherein the intraocular route is an intravitreal injection.

7. Pharmaceutical composition according to anyone of Claims **1** to **6,** wherein the composition is injected at most once a week, preferably once every two weeks, more preferably once a month, even more preferably once every two months.

8. Pharmaceutical composition according to anyone of Claims **1** to **7,** wherein the composition is packaged in the form of unit dose.

9. Pharmaceutical composition according to anyone of Claims **1** to **8**, wherein the composition is sterile.

10. Pharmaceutical composition according to Claim **8** or Claim **9**, wherein the unit dose is a disposable syringe.

11. Pharmaceutical composition according to anyone of Claims **1** to **10,** wherein the pathological neovascularization-related condition may be uveitis, choroiditis, retinochoroiditis, chorioretinitis, retinal degeneration, AMD, retinal detachment, retinal neovascularisation, proliferative vitreoretinopathy, retinopathy of prematurity (ROP), diabetic retinopathy, posterior segment trauma, retinal vascular pathologies, endophthalmitis, macular edema, diabetic retinopathy, inflammatory pathologies of the retina, systemic pathologies with implications for the retina.

12. Method for treating a pathological neovascularization-related condition of the interior of the eye in a subject in need thereof comprising administering to the subject a therapeutically effective amount of a pharmaceutical composition according to anyone of claims **1** to **11** by intraocular route.


SEQUENCE LISTING

<110>  GENE SIGNAL INTERNATIONAL SA
       Al-Mahmood, Salman et al.

<120>  INJECTABLE PHARMACEUTICAL COMPOSITION FOR PREVENTING, STABILISING
        AND/OR INHIBITING PATHOLOGICAL NEOVASCULARIZATION-RELATED
       CONDITIONS

<130>  CV/104

<160>  20

<170>  PatentIn version 3.5

<210>  1
<211>  25
<212>  DNA
<213>  artificial

<220>
<223>  IRS-1 antisens

<400>  1
tctccggagg gctcgccatg ctgct                                            25


<210>  2
<211>  25
<212>  DNA
<213>  artificial

<220>
<223>  IRS-1 antisens

<400>  2
tatccggagg gctcgccatg ctgct                                            25


<210>  3
<211>  24
<212>  DNA
<213>  artificial

<220>
<223>  IRS-1 antisens

<400>  3
tctccggagg gctcgccatg ctgc                                             24


<210>  4
<211>  23
<212>  DNA
<213>  artificial

<220>
<223>  IRS-1 antisens

<400>  4
tctccggagg gctcgccatg ctg                                              23


<210>  5
<211>  22
<212>  DNA
<213>  artificial

<220>
<223>  IRS-1 antisens

<400>  5
tctccggagg gctcgccatg ct                                                22

<210>  6
<211>  21
<212>  DNA
<213>  artificial

<220>
<223>  IRS-1 antisens

<400>  6
tctccggagg gctcgccatg c                                                 21

<210>  7
<211>  20
<212>  DNA
<213>  artificial

<220>
<223>  IRS-1 antisens

<400>  7
tctccggagg gctcgccatg                                                   20

<210>  8
<211>  19
<212>  DNA
<213>  artificial

<220>
<223>  IRS-1 antisens

<400>  8
tctccggagg gctcgccat                                                    19

<210>  9
<211>  24
<212>  DNA
<213>  artificial

<220>
<223>  IRS-1 antisens

<400>  9
ctccggaggg ctcgccatgc tgct                                              24

<210>  10
<211>  23
<212>  DNA
<213>  artificial

<220>
<223>  IRS-1 antisens

<400>  10
tccggagggc tcgccatgct gct                                               23

<210>  11

<211>  22
<212>  DNA
<213>  artificial

<220>
<223>  IRS-1 antisens

<400>  11
ccggagggct cgccatgctg ct                                                22


<210>  12
<211>  21
<212>  DNA
<213>  artificial

<220>
<223>  IRS-1 antisens

<400>  12
cggagggctc gccatgctgc t                                                 21


<210>  13
<211>  20
<212>  DNA
<213>  artificial

<220>
<223>  IRS-1 antisens

<400>  13
ggagggctcg ccatgctgct                                                   20


<210>  14
<211>  19
<212>  DNA
<213>  artificial

<220>
<223>  IRS-1 antisens

<400>  14
gagggctcgc catgctgct                                                    19


<210>  15
<211>  18
<212>  DNA
<213>  artificial

<220>
<223>  IRS-1 antisens

<400>  15
agggctcgcc atgctgct                                                     18


<210>  16
<211>  16
<212>  DNA
<213>  artificial

<220>
<223>  IRS-1 antisens

<400>  16

ggctcgccat gctgct                                                    16


<210>  17
<211>  15
<212>  DNA
<213>  artificial

<220>
<223>  IRS-1 antisens

<400>  17
gctcgccatg ctgct                                                     15


<210>  18
<211>  14
<212>  DNA
<213>  artificial

<220>
<223>  IRS-1 antisens

<400>  18
ctcgccatgc tgct                                                      14


<210>  19
<211>  13
<212>  DNA
<213>  artificial

<220>
<223>  IRS-1 antisens

<400>  19
tcgccatgct gct                                                       13


<210>  20
<211>  12
<212>  DNA
<213>  artificial

<220>
<223>  IRS-1 antisens

<400>  20
cgccatgctg ct                                                        12